# FASCICULE DE BREVET EUROPEEN

(11) **EP 3 518 818 B1**
(45) Date de publication et mention de la délivrance du brevet: **30.12.2020**
(21) Numéro de dépôt: 17786975.7
(22) Date de dépôt: 03.10.2017
(51) Int. Cl.: A61C 8/00, A61B 17/80

(54) **PLAQUE MANDIBULAIRE ET KIT DE MISE EN PLACE**
BANDAGE UND KIT ZUR INSTALLATION DAVON
JAW PLATE AND KIT FOR INSTALLING SAME

(30) Priorité: 03.10.2016 FR 1659488
(43) Date de publication de la demande: 07.08.2019
(73) Titulaire: Global D, 69530 Brignais (FR)
(72) Inventeur: CASIMIRO, Roméo, 69230 Saint Genis Laval (FR); BEDHET, Nicolas, 35000 Rennes (FR)
(74) Mandataire: Corret, Hélène
(86) Numéro de dépôt international: PCT/FR2017/052718
(87) Numéro de publication internationale: WO 2018/065725

(56) Documents cités:
- WO-A1-01/30249
- US-A1- 2007 259 306
- US-A1- 2010 016 900

## Description

La présente invention concerne le domaine technique des plaques de fixation pour des os mandibulaires.

Une mandibule peut être fracturée du fait d'un traumatisme. Elle peut également être séparée en deux valves dans le cadre d'actes de chirurgie orthognatique. Ces actes ont pour objet d'améliorer l'équilibre facial et/ou d'assurer une meilleure occlusion des arcades dentaires.

Dans tous les cas, il est nécessaire de réduire la fracture et de maintenir en position les deux parties de la mandibule concernée, jusqu'à leur soudure grâce à la croissance osseuse.

Les plaques de fixation d'os mandibulaires sont communément utilisées dans ce but.

On peut notamment citer le document US 9066767 qui décrit une telle plaque, associée à une pluralité de moyens de fixation.

Le document WO 01/30249 décrit deux plaques reliées l'une à l'autre par un mécanisme d'assemblage qui est réglable.

Le document US 2007/259306 décrit une plaque orthodontique et non une plaque de fixation d'os mandibulaires.

Le document US 2010/016900 décrit une plaque anatomique selon le préambule de la revendication 1.

Une telle plaque présente une forme différente selon qu'elle est destinée à être placée du côté droit de la mandibule (plaque droite) ou du côté gauche (plaque gauche). Ces formes sont en miroir l'une de l'autre.

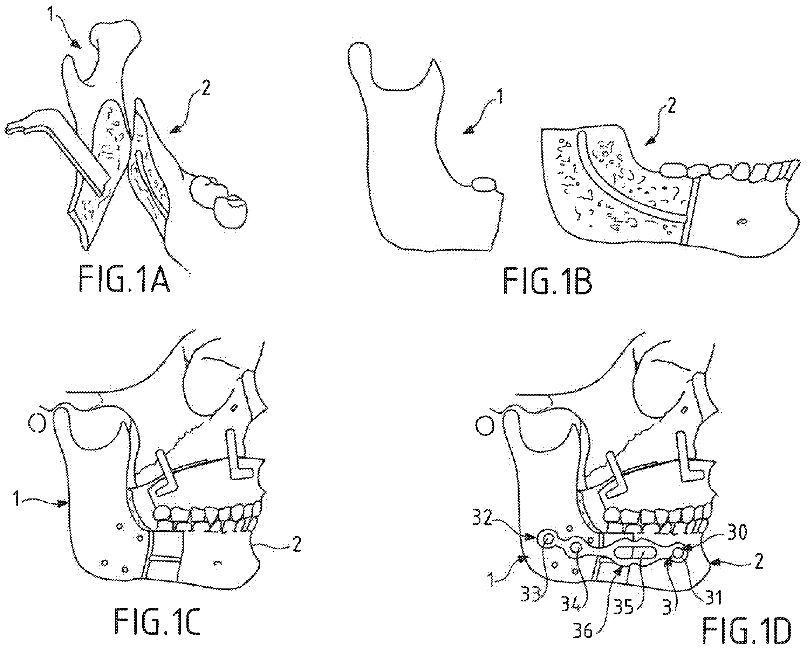

Un procédé classique de mise en place d'une plaque dans le cadre de chirurgies orthognatiques sera maintenant décrit en référence aux figures 1A à 1D.

La figure 1A illustre la séparation, réalisée par le praticien, de la mandibule en deux valves : la valve externe 1 et la valve interne 2 (la plus proche du centre de la bouche). Ces deux valves sont illustrées, vues de côté sur la figure 1B.

Les deux valves 1 et 2 sont ensuite placées par le praticien dans le positionnement voulu. Dans l'exemple illustré à la figure 1C, les deux valves sont écartées l'une de l'autre.

Le praticien utilise ensuite une plaque 3 pour maintenir ensemble les deux valves 1 et 2.

Cette plaque 3 présente une forme allongée, avec une partie avant 30 dans laquelle est pratiqué un trou 31, une partie arrière 32 dans laquelle sont pratiqués deux trous 33 et 34 et une partie centrale 36, entre la partie avant 30 et la partie arrière 32, dans laquelle est réalisé un trou de forme oblongue 35.

La majorité des plaques disponibles sur le marché sont planes et le praticien doit donc plier la plaque 3 pour l'adapter au mieux à la morphologie du patient. Cette mise en forme crée des contraintes sur la plaque qui peuvent générer ensuite des déplacements de la plaque après sa mise en place sur le patient.

Dans un premier temps, le praticien fixe la plaque 3 sur la valve externe 1 grâce à des vis qui passent à travers les trous postérieurs 33 et 34. Il ajuste ensuite la position de la plaque, et donc de la valve externe qui en est solidaire, et place une vis à travers le trou oblong 35. Enfin, il fixe définitivement la plaque en insérant une vis dans l'os, à travers le trou antérieur 31.

Ainsi une fois les vis placées dans les trous postérieurs 33 et 34, le praticien peut avoir besoin de repositionner la plaque, afin de positionner de façon appropriée la valve externe 1 par rapport à la valve interne 2.

Il est alors obligé d'utiliser une pince ou un tournevis pour manipuler la plaque, en venant par exemple en prise sur le trou avant 31. Or, le praticien ne dispose que de peu d'espace et sa liberté de mouvement est donc limitée.

C'est pourquoi les risques de blesser le patient lors de ce positionnement sont importants.

L'invention a pour objet de pallier ces inconvénients en proposant une plaque de conception spécifique permettant sa manipulation par un ancillaire, sans risque de blessure du patient.

L'invention concerne donc une plaque de fixation d'os mandibulaires, de forme allongée et s'étendant principalement selon une direction longitudinale L, avec une face interne destinée à venir en contact avec les os mandibulaires et une face externe opposée, ladite plaque comportant un côté avant et un côté arrière, reliés par un côté inférieur et un côté supérieur, et dans laquelle sont percés au moins trois orifices pour des moyens de fixation dans les os mandibulaires, au moins un orifice à l'arrière de la plaque, un orifice à l'avant de la plaque et un orifice de forme oblongue situé entre ledit au moins un orifice arrière et l'orifice avant et s'étendant sensiblement selon la direction longitudinale L, caractérisée en ce qu'elle comporte une encoche prévue dans le côté avant de la plaque ou dans son côté supérieur entre le côté avant jusqu'au-dessus de l'extrémité arrière de l'orifice oblong, cette encoche étant agencée pour coopérer avec l'extrémité d'un ancillaire.

Dans des modes de réalisation avantageux, on a de plus recours à l'une et/ou à l'autre des dispositions suivantes :
- ladite encoche s'étend sensiblement dans la direction longitudinale L ;
- la plaque comporte, sur sa face interne, un premier évidement dans le prolongement du fond de l'encoche ;
- le premier évidement délimite un pan coupé ;
- la plaque comporte également un deuxième évidement sur la face externe de la plaque ;
- le deuxième évidement délimite également un pan coupé, le premier et le deuxième évidements définissant un profil en V, le fond du V étant situé au fond de l'encoche ;
- la plaque est pliée, vers sa face interne, autour d'au moins une ligne de pliage.

L'invention concerne également un kit comprenant une plaque de fixation d'os mandibulaires selon la revendication 1 ou 2 et un ancillaire pour la mise en place de ladite plaque sur lesdits os, ledit ancillaire comprenant une tige rigide avec une extrémité proximale et une extrémité distale, cette dernière étant apte à s'insérer dans l'encoche de la plaque et comprenant un évidement pour coopérer avec le fond de l'encoche, en butée ou en encastrement et venir en prise avec ladite plaque.

Dans des modes de réalisation avantageux, on a de plus recours à l'une et/ou à l'autre des dispositions suivantes :
- la plaque comportant sur sa face interne un premier évidement dans le prolongement du fond de l'encoche, une partie de l'extrémité distale de l'ancillaire est apte à s'insérer dans ledit premier évidement ;
- ledit premier évidement délimite un pan coupé reliant la face interne et la face externe de la plaque et l'extrémité distale de l'ancillaire présente la forme d'une fourche à deux dents dont les faces intérieures forment un V, l'une des dents étant susceptible de s'insérer dans ledit premier évidement ;
- la plaque comporte un deuxième évidement sur sa face externe délimitant un pan coupé, le premier et le deuxième évidements définissant un profil en V correspondant à celui formé par la fourche de l'extrémité distale de l'ancillaire.

L'invention concerne également un procédé de mise en place d'une plaque de fixation sur la valve externe et la valve interne d'une mandibule avec un kit selon l'invention, le procédé comprenant les étapes suivantes :
- positionner la plaque entre la valve externe et la valve interne de la mandibule,
- insérer des moyens de fixation dans la valve externe au travers dudit au moins un orifice arrière,
- insérer l'extrémité distale de l'ancillaire dans l'encoche de la plaque pour coopérer avec le fond de l'encoche, en butée ou en encastrement, et venir en prise avec ladite plaque,
- exercer une force de poussée ou de tirage sur ladite plaque grâce à l'ancillaire, pour donner à ladite plaque le positionnement souhaité,
- insérer des moyens de fixation dans la valve interne à travers l'orifice oblong puis l'orifice avant de la plaque.

L'invention sera mieux comprise et d'autres buts, avantages et caractéristiques de celle-ci apparaitront plus clairement à la lecture de la description qui suit et qui est faite au regard de dessins annexés sur lesquels :
Les figures 1A à 1D illustrent la mise en place sur un patient d'une plaque pour os mandibulaires selon l'état de la technique.
La figure 2 illustre une vue schématique en plan d'un exemple de réalisation d'une plaque droite selon l'invention.
La figure 3 illustre une vue en coupe selon A-A de la figure 2.
La figure 4 est une vue schématique d'un exemple de plaque selon l'invention sur laquelle sont illustrés des axes de pliage.
La figure 5 est une vue en perspective d'un exemple de plaque selon l'invention.
La figure 6 est une vue en perspective d'un exemple de réalisation d'un ancillaire selon l'invention.
La figure 6A est une vue agrandie de l'extrémité distale de l'ancillaire illustré à la figure 6.
Les figures 7A à 7F illustrent les différentes étapes de mise en place sur une mandibule d'un exemple de réalisation d'une plaque selon l'invention.

Les éléments communs aux différentes figures seront désignés par les mêmes références.

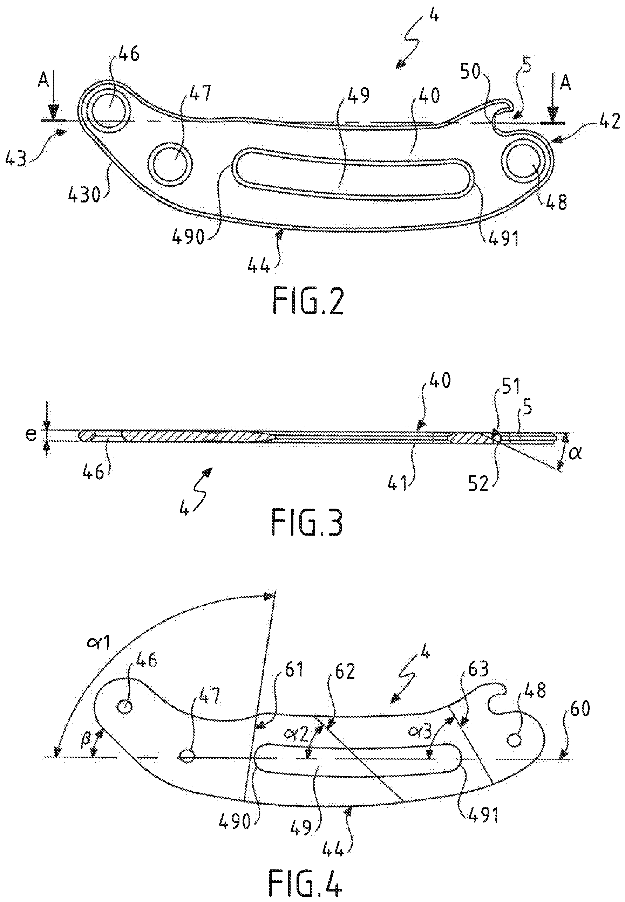

Il est maintenant fait référence à la figure 2 qui décrit un exemple de réalisation d'une plaque de fixation d'os mandibulaires selon l'invention. Cette plaque est destinée à être placée du côté droit de la mandibule d'un patient (plaque droite). Bien entendu, la structure d'une plaque gauche se déduit de celle qui va être décrite, ces deux plaques étant en miroir l'une de l'autre.

Cette plaque 4 comporte une forme allongée et elle s'étend principalement selon une direction longitudinale L, matérialisée par un axe longitudinal médian de la plaque.

Cette plaque 4 définit une face interne 40 destinée à venir en contact avec les os mandibulaires et une face externe 41 opposée.

Son contour périphérique comprend un côté avant 42 et un côté arrière 43 qui sont reliés l'un à l'autre par un côté inférieur 44 et un côté supérieur 45.

Ce contour définit le bord de la plaque.

Dans l'exemple illustré sur la figure 2, le côté arrière 43 présente la forme d'une courbe dont la partie 430 rejoignant le côté inférieur 44 fait un angle β avec l'axe longitudinal médian L de la plaque (voir figure 4).

Cet angle β est ici d'environ 44°, sans que l'invention soit limitée à cette valeur.

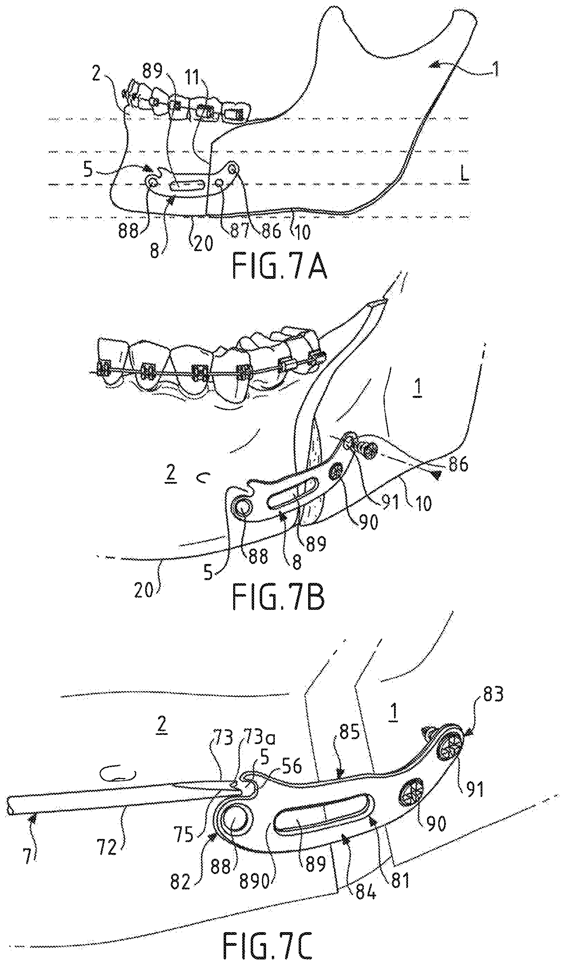

Dans cette plaque 4, sont pratiqués quatre orifices ou trous traversants qui sont destinés à recevoir des moyens de fixation dans les os mandibulaires, comme cela sera décrit plus en détail au regard des figures 7A à 7F.

A l'arrière de la plaque, c'est-à-dire dans la région définie par le côté arrière 43 sont réalisés un premier et un deuxième orifice arrière 46 et 47 qui sont écartés l'un de l'autre selon la direction longitudinale L.

Un autre orifice 48 est prévu à l'avant de la plaque 4, c'est-à-dire à proximité du côté avant 42 de la plaque.

Ces trois orifices 46 à 48 sont sensiblement de forme ronde.

Un quatrième orifice 49 est prévu dans la région centrale de la plaque, entre le deuxième orifice arrière 47 et l'orifice avant 48. On comprend que l'axe L est situé sensiblement à mi-hauteur de cette région centrale.

Cet orifice 49 présente une forme oblongue qui s'étend sensiblement selon la direction longitudinale L.

Ces orifices sont sensiblement alignés sur un arc de cercle pour assurer une meilleure répartition des contraintes, lorsque la plaque est fixée sur les valves de la mandibule.

L'invention n'est pas limitée à cet exemple de réalisation et l'on pourrait prévoir un seul orifice arrière avec un orifice oblong de longueur plus importante afin de pouvoir insérer dans la valve externe des moyens de fixation passant à travers cet orifice arrière et l'orifice oblong.

La figure 2 montre que la plaque 4 comporte également une encoche 5 qui est réalisée dans le côté avant 42 de la plaque.

Cette encoche s'étend de préférence sensiblement selon la direction longitudinale L, de façon à faciliter sa coopération avec un ancillaire et sa manipulation par celui-ci, comme cela sera décrit par la suite.

En pratique, l'inclinaison de l'encoche par rapport à la direction longitudinale L est comprise entre 0° et 20°.

L'invention n'est pas limitée à ce mode de réalisation et cette encoche pourrait également être réalisée dans le côté supérieur 45 de la plaque 4 et ce, dans toute région située entre le côté avant 42 et l'extrémité arrière 490 de l'orifice oblong 49.

L'encoche 5 est une découpe réalisée dans la plaque et elle forme une cavité ouverte vers l'extérieur dont le contour est arrondi dans les exemples illustrés.

Cependant, l'invention n'est pas limitée à ce mode de réalisation et l'encoche pourrait présenter un contour différent.

Elle peut être définie de façon générale par deux parois sensiblement longitudinales reliées par un fond s'étendant sensiblement transversalement.

Cette plaque 4 est réalisée en un matériau rigide implantable, tel que du Titane, notamment de Grade 2, 4, 5 ou 23.

Par ailleurs, la plaque selon l'invention peut présenter des longueurs différentes pour mieux s'adapter à chaque patient.

Il est maintenant fait référence à la figure 3 qui permet d'illustrer, vue en coupe, l'encoche 5.

La figure 3 montre l'épaisseur e de la plaque 4 qui est typiquement égale à environ 0,8 mm et notamment comprise entre 0,6 mm et 1,5 mm.

La figure 3 montre que, dans ce mode préféré de réalisation, le fond 50 de l'encoche se prolonge, sur la face interne 40 de la plaque, par un premier évidement 51.

Cet évidement délimite un pan coupé 52 qui s'étend depuis la face interne 40 jusqu'à la face externe 41 de la plaque 4, selon un angle α. Le pan coupé 52 se termine à l'extrémité de la face extérieure 41 de la plaque.

Cet angle α est, dans l'exemple illustré, égal à 25° et il est typiquement compris entre 10° et 45°.

L'invention n'est pas limitée à ce mode de réalisation. En particulier, la plaque 4 pourrait également comporter un deuxième évidement, cette fois sur la face externe 41 de la plaque.

De préférence, ce deuxième évidement peut délimiter un pan coupé, de telle sorte que les deux évidements définissent un profil en V, le fond du V étant situé au niveau du fond 50 de l'encoche 5.

L'intérêt de cet évidement 51 sera décrit dans la suite de la description.

Il est maintenant fait référence à la figure 4 qui illustre les différents axes selon lesquels la plaque est pliée lorsqu'elle est découpée dans une plaque de titane, pour lui donner une forme s'adaptant à la majorité des types de mandibules et de positionnements relatifs de la valve interne et de la valve externe.

Cette conformation anatomique donnée préalablement à la plaque permet de limiter les contraintes sur le matériau et de mieux garantir le maintien de la position et du déplacement souhaités.

Il convient de noter qu'un pliage selon au moins une ligne de pliage peut donner à la plaque une première conformation, qui sera ensuite éventuellement ajustée par le praticien.

La figure 4 illustre quatre lignes de pliage 60 à 63.

La première ligne de pliage 60 correspond à l'axe longitudinal médian L de la plaque et elle s'étend donc dans la direction longitudinale de la plaque.

Les trois autres lignes de pliage 61 à 63 sont inclinées par rapport à la première ligne de pliage 60.

La deuxième ligne de pliage 61 est inclinée d'un angle α₁ par rapport à la première ligne de pliage 60.

Cet angle α₁ est, dans cet exemple, égal à environ 95°. L'invention n'est cependant pas limitée à cet exemple et cet angle α₁ peut-être typiquement compris entre 45° et 135°.

La troisième ligne de pliage 62 est inclinée d'un angle α₂ par rapport à la première ligne de pliage 60 qui est ici d'environ 45°. Cet angle α₂ peut typiquement être compris entre 30° et 90°.

Enfin, la quatrième ligne de pliage 63 fait un angle α₃ avec la première ligne de pliage 60 qui est ici égal à 60° et qui est typiquement compris entre 30° et 90°.

On note que la deuxième ligne de pliage 61 est située entre le deuxième orifice arrière 47 et l'extrémité arrière 490 de l'orifice oblong 49, tandis que la quatrième ligne de pliage 63 est située entre l'orifice avant 48 et l'extrémité avant 491 du trou oblong 49.

Enfin, la troisième ligne de pliage 63 passe par la partie centrale de l'orifice oblong 49.

Les courbures données à la plaque 4 sont les suivantes :
Tout d'abord, la plaque est pliée autour de la troisième ligne de pliage 62 depuis la face externe vers la face interne, de façon à lui donner un profil concave et assurer ainsi une bonne adaptation de la plaque aux os mandibulaires.

L'angle de pliage autour de la troisième ligne 62 est généralement compris entre 10° et 30° et notamment égal à par exemple 23° ou 25°.

Le pliage de la plaque 4 autour des deuxième et quatrième lignes de pliage 61 et 63 est également réalisé depuis la face externe 40 de la plaque vers la face interne.

Ce pliage des deux zones d'extrémité de la plaque 4 permet d'éviter les contraintes au niveau des articulations temporo-mandibulaires.

L'angle de pliage autour de la deuxième ligne 61 est généralement compris entre 5° et 20° et peut être égal à 13° ou 18° par exemple.

L'angle de pliage autour de la quatrième ligne 63 est, quant à lui, généralement compris entre 10° et 40° et peut par exemple être égal à 20° ou 30°.

Enfin, la plaque 4 est vrillée autour de la première ligne de pliage 60 pour obtenir un décalage angulaire généralement compris entre 5° et 40°.

Cette conformation spiroïdale suivant l'axe longitudinal L de la plaque permet également d'éviter les contraintes au niveau des articulations temporo-mandibulaires.

A titre de variante et dans le même but, la plaque 4 est pliée autour de la première ligne de pliage toujours depuis la face externe vers la face interne et selon un angle généralement compris entre 5° et 20° et par exemple égal à 10°.

De façon générale, le pliage autour de chacune des lignes de pliage est réalisé pour ménager une portion courbe au niveau de la ligne de pliage dont le rayon de courbure est notamment compris entre 3 et 20 mm.

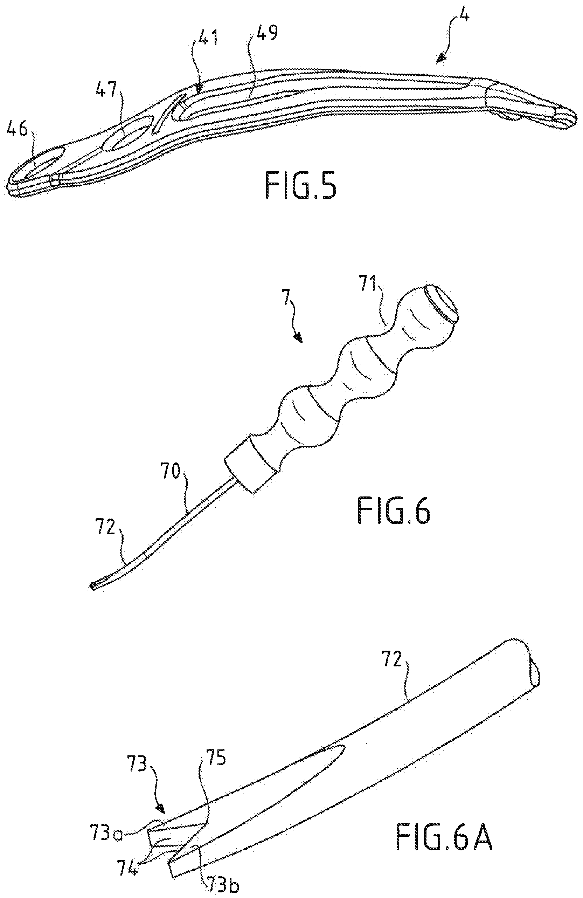

La figure 5 illustre en perspective la plaque 4 et montre ainsi l'effet des différents pliages réalisés sur la plaque 4.

La plaque peut également être obtenue directement avec la conformation illustrée à la figure 5 lorsqu'on met en œuvre des procédés de fabrication soustractive ou additive.

Il est maintenant fait référence aux figures 6 et 6A qui illustrent un exemple de réalisation d'un ancillaire selon l'invention.

Cet ancillaire 7 comporte une tige rigide 70 dont l'extrémité proximale est munie de moyens de préhension 71, ces moyens permettant au praticien de saisir et manipuler facilement l'ancillaire 7.

A son extrémité distale, la tige 70 comporte une partie 72 qui se termine par une fourche 73 à deux dents 73a, 73b dont les faces intérieures 74 forment un V. Le fond 75 du V est en retrait à l'intérieur de la partie courbée 72, un évidement est ainsi formé.

Les figures 6 et 6A montrent une partie 72 incurvée mais l'invention n'est pas limitée à ce mode de réalisation.

Il est maintenant fait référence aux figures 7A à 7F au regard desquelles va être décrite la mise en place d'une plaque selon l'invention sur les deux valves d'une mandibule.

La plaque 8 illustrée sur ces figures est destinée à être placée sur le côté gauche de la mandibule (plaque gauche).

Cette plaque 8 ne sera pas de nouveau décrite en détail.

Les références numériques seront similaires à celles utilisées pour la plaque 4 illustrée à la figure 2.

La figure 7A illustre une valve externe 1 et une valve interne 2.

Le praticien positionne la plaque 8 à cheval sur les deux valves 1 et 2.

De préférence, la plaque est positionnée de telle sorte que son axe longitudinal médian est sensiblement parallèle aux bords basilaires 10, 20 des valves 1 et 2. Elle est également positionnée sensiblement au tiers de la hauteur de la valve interne 2, comptée depuis le bord basilaire 10, 20 de chaque valve.

Cependant, si la hauteur de la mandibule est faible, la plaque peut être positionnée plus bas, à la limite des bords basilaires.

Par ailleurs, la plaque 8 est positionnée de telle sorte que le centre du deuxième orifice arrière 87 est situé à au moins 4 mm du trait d'ostéotomie 11.

La figure 7B illustre l'étape suivante dans laquelle le praticien insère dans la valve externe 1, un premier moyen de fixation 90 en passant dans le deuxième orifice arrière 87 puis un deuxième moyen de fixation 91 en passant dans le premier orifice arrière 86. Le praticien s'assure que l'axe longitudinal médian L de la plaque 8 est toujours sensiblement parallèle aux bords basilaires 10, 20 des valves.

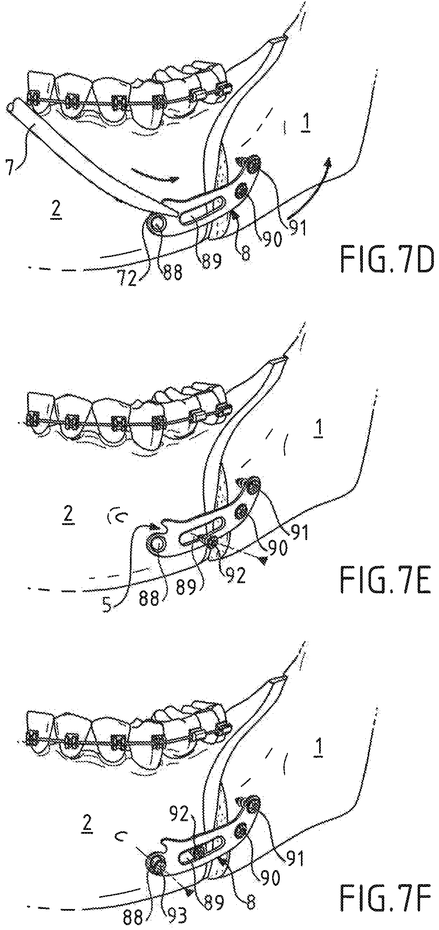

La figure 7D montre l'étape suivante dans laquelle le praticien insère l'ancillaire 7 dans l'encoche 5 de la plaque 8.

La figure 7C illustre plus précisément l'orientation de l'extrémité distale 72 de l'ancillaire 7 lorsque le praticien l'insère dans l'encoche 5. Cette figure montre que la fourche 73 est située dans un plan sensiblement perpendiculaire au fond 50 de l'encoche mais que l'ancillaire s'étend dans le plan défini par la plaque.

Ainsi, le fond 75 du V formé par la fourche 73 peut venir en contact avec le fond 50 de l'encoche, tandis que la dent 73a vient s'insérer dans l'évidement 51 de la plaque, situé sur la face interne de la plaque, opposée à sa face externe 81.

La figure 7D montre l'ancillaire 7 avec son extrémité distale 72 coopérant avec l'encoche et plus particulièrement le fond 50 de l'encoche.

Grâce à cette coopération entre l'encoche et l'extrémité distale 72 de l'ancillaire, celui-ci vient en butée avec la plaque. Cette coopération assure un blocage des mouvements de l'ancillaire 7 dans le plan frontal et également dans le plan sagittal lorsque le praticien exerce une poussée vers l'arrière de la bouche. L'ancillaire peut être manipulé en toute sécurité par le chirurgien sans risque de dérapage et donc de blessure du patient, lorsque le praticien exerce une force de poussée vers l'arrière.

L'ancillaire peut être dégagé de l'encoche par un mouvement dans le plan sagittal et le plan horizontal et vers l'avant de la bouche.

De plus, dans la mesure où la dent 73a est insérée dans l'évidement 51 de l'encoche formant un pan coupé 52, du côté de sa face interne, aucune surépaisseur n'est générée, ce qui contribue également à éviter toute blessure du patient. Cette insertion assure de plus un blocage des mouvements de l'ancillaire 7 dans le plan transversal.

La figure 7D montre que, grâce à la coopération entre l'ancillaire 7 et la plaque 8, le praticien peut agir sur la plaque 8 et assurer son bon positionnement par rapport à la valve interne. Dans l'exemple illustré sur les figures, les deux valves 1 et 2 sont écartées l'une de l'autre.

La figure 7E illustre l'étape suivante dans laquelle un troisième moyen de fixation 92 est fixé dans la valve interne 2 en passant par l'orifice oblong 89.

Son positionnement à l'intérieur de l'orifice 89 est choisi en fonction du positionnement de la plaque souhaité par le praticien.

La figure 7F illustre la dernière étape de mise en place de la plaque 8 sur les valves 1 et 2, étape dans laquelle un quatrième moyen de fixation 93 est fixé sur la valve interne 2, à travers l'orifice avant 88.

De façon préférée, deux moyens de fixation sont insérés, à travers la plaque, dans la valve interne et dans la valve externe, de façon à éviter tout mouvement de rotation entre la plaque et l'une des valves, après mise en place de la plaque, lors de la période de consolidation osseuse.

Dans les exemples illustrés et décrits, les moyens permettant la coopération entre l'ancillaire et la plaque permettent essentiellement au praticien d'exercer une force de poussée sur la plaque vers l'arrière, le fond de la fourche venant en butée avec le fond de l'encoche.

Cependant, l'invention n'est pas limitée à ce mode de réalisation et l'on pourrait également envisager des moyens permettant d'encastrer et de solidariser l'ancillaire et la plaque, de façon à ce que le praticien puisse également exercer une force de tirage vers l'avant sans entrainer la désolidarisation de l'ancillaire et de la plaque.

## Revendications

1. Plaque de fixation d'os mandibulaires, de forme allongée et s'étendant principalement selon une direction longitudinale L, avec une face interne (40) destinée à venir en contact avec les os mandibulaires et une face externe opposée (41, 81), ladite plaque comportant un côté avant (42, 82) et un côté arrière (43, 83), reliés par un côté inférieur (44, 84) et un côté supérieur (45, 85), et dans laquelle sont percés au moins trois orifices (46 à 49 ; 86 à 89) pour des moyens de fixation (90 à 93) dans les os mandibulaires (1, 2), au moins un orifice (46, 47 ; 86, 87) à l'arrière de la plaque, un orifice (48, 88) à l'avant de la plaque et un orifice (49, 89) de forme oblongue situé entre ledit au moins un orifice arrière (47) et l'orifice avant et s'étendant sensiblement selon la direction longitudinale L, **caractérisée en ce qu'**elle comporte une encoche (5) qui s'étend sensiblement dans la direction longitudinale L et comprend deux parois sensiblement longitudinales reliées par un fond s'étendant sensiblement transversalement, dans le côté avant (42, 82) de la plaque ou dans son côté supérieur (45, 85) entre le côté avant jusqu'au-dessus de l'extrémité arrière (490, 890) de l'orifice oblong (49, 89), cette encoche étant agencée pour coopérer avec l'extrémité distale d'un ancillaire.

2. Plaque selon la revendication 1, **caractérisée en ce que** l'inclinaison de ladite encoche (5) par rapport à la direction longitudinale L est comprise entre 0 et 20°.

3. Plaque selon la revendication 1 ou 2, **caractérisée en ce qu'**elle comporte, sur sa face interne (40), un premier évidement (51) dans le prolongement du fond (50) de l'encoche.

4. Plaque selon la revendication 3, **caractérisée en ce que** le premier évidement (51) délimite un pan coupé.

5. Plaque selon la revendication 3 ou 4, **caractérisée en ce qu'**elle comporte également un deuxième évidement sur la face externe (41, 81) de la plaque.

6. Plaque selon la revendication 5, **caractérisé en ce que** le deuxième évidement délimite également un pan coupé, le premier et le deuxième évidements définissant un profil en V, le fond du V étant situé au fond (50) de l'encoche.

7. Plaque selon l'une des revendications 1 à 6, **caractérisée en ce qu'**elle est pliée, vers sa face interne, autour d'au moins une ligne de pliage (60 à 63) .

8. Kit comprenant une plaque de fixation d'os mandibulaires (4, 8) selon la revendication 1 ou 2 et un ancillaire (7) pour la mise en place de ladite plaque sur lesdits os, ledit ancillaire comprenant une tige (70) rigide avec une extrémité proximale et une extrémité distale (72), cette dernière étant apte à s'insérer dans l'encoche (5) de la plaque et comprenant un évidement pour coopérer avec le fond (50) de l'encoche, en butée ou en encastrement, et venir en prise avec ladite plaque.

9. Kit selon la revendication 8, dans lequel la plaque comporte sur sa face interne (40) un premier évidement (51) dans le prolongement du fond (50) de l'encoche, une partie de l'extrémité distale (72) de l'ancillaire étant apte à s'insérer dans ledit premier évidement.

10. Kit selon la revendication 9, dans lequel ledit premier évidement (51) délimite un pan coupé (52) reliant la face interne et la face externe de la plaque et l'extrémité distale de l'ancillaire présente la forme d'une fourche (73) à deux dents (73a, 73b) dont les faces intérieures (74) forment un V, l'une des dents étant susceptible de s'insérer dans ledit premier évidement (51).

11. Kit selon la revendication 10, dans lequel la plaque comporte un deuxième évidement sur sa face externe délimitant un pan coupé, le premier et le deuxième évidements définissant un profil en V correspondant à celui formé par la fourche de l'extrémité distale de l'ancillaire.

## Patentansprüche

1. Platte zur Fixierung von Unterkieferknochen, welche länglich ist und sich im Wesentlichen in einer Längsrichtung L erstreckt, mit einer Innenfläche (40), die dazu bestimmt isst, mit den Unterkieferknochen in Kontakt zu kommen, und einer entgegengesetzten Außenfläche (41, 81), wobei die Platte eine Vorderseite (42, 82) und eine Rückseite (43, 83) aufweist, welche durch eine Unterseite (44, 84) und eine Oberseite (45, 85) verbunden sind, und in der mindestens drei Öffnungen (46 bis 49; 86 bis 89) für Mittel (90 bis 93) zur Fixierung in den Unterkieferknochen (1, 2) gebohrt sind - mindestens eine Öffnung (46, 47; 86, 87) im hinteren Bereich der Platte, eine Öffnung (48, 88) im vorderen Bereich der Platte und eine längliche Öffnung (49, 89) zwischen der mindestens einen hinteren Öffnung (47) und der vorderen Öffnung, welche sich im Wesentlichen in der Längsrichtung L erstreckt,
**dadurch gekennzeichnet,**
**dass** sie eine Einkerbung (5) aufweist, die sich im Wesentlichen in der Längsrichtung L erstreckt und zwei im Wesentlichen länglichen Wände aufweist, die durch einen Boden verbunden sind, welcher sich im Wesentlichen quer erstreckt, in der Vorderseite (42, 82) der Platte oder in ihrer Oberseite (45, 25, 85) zwischen der Vorderseite bis über dem hinteren Ende (490, 890) der länglichen Öffnung (49, 89), wobei diese Einkerbung angeordnet ist, um mit dem distalen Ende eines Hilfsinstruments zusammenzuwirken.

2. Platte nach Anspruch 1,
**dadurch gekennzeichnet,**
**dass** die Neigung der Einkerbung (5) relativ zur Längsrichtung L zwischen 0 und 20° liegt.

3. Platte nach Anspruch 1 oder 2,
**dadurch gekennzeichnet,**
**dass** sie auf ihrer Innenfläche (40) eine erste Ausnehmung (51) in der Verlängerung des Bodens (50) der Einkerbung aufweist.

4. Platte nach Anspruch 3,
**dadurch gekennzeichnet,**
**dass** die erste Ausnehmung (51) eine Abschrägung begrenzt.

5. Platte nach Anspruch 3 oder 4,
**dadurch gekennzeichnet,**
**dass** sie ferner eine zweite Ausnehmung auf der Außenfläche (41, 81) der Platte aufweist.

6. Platte nach Anspruch 5,
**dadurch gekennzeichnet,**
**dass** die zweite Ausnehmung ebenfalls eine Abschrägung begrenzt, wobei die erste und die zweite Ausnehmung ein V-Profil definieren, wobei der Boden des V sich auf dem Boden (50) der Einkerbung befindet.

7. Platte nach einem der Ansprüche 1 bis 6,
**dadurch gekennzeichnet,**
**dass** sie zu ihrer Innenfläche hin um mindestens eine Knicklinie (60 bis 63) geknickt ist.

8. Set umfassend eine Platte zur Fixierung von Unterkieferknochen (4, 8) nach Anspruch 1 oder 2,
und ein Hilfsinstrument (7) zum Anbringen der Platte auf die Knochen, wobei das Hilfsinstrument einen starren Schaft (70) mit einem proximalen und einem distalen Ende (72) aufweist, wobei Letztere geeignet ist, in die Einkerbung (5) der Platte hineinzupassen, und eine Ausnehmung aufweist, um mit dem Boden (50) der Einkerbung mit Anschlag oder mit Einbau zusammenzuwirken und mit der Platte in Eingriff zu gelangen.

9. Set nach Anspruch 8,
bei dem die Platte auf ihrer Innenfläche (40) eine erste Ausnehmung (51) in der Verlängerung des Bodens (50) der Einkerbung aufweist, wobei ein Teil des distalen Endes (72) des Hilfsinstruments geeignet ist, in die erste Ausnehmung zu greifen.

10. Set nach Anspruch 9,
bei dem die erste Ausnehmung (51) eine Abschrägung (52) begrenzt, welche die Innenfläche und die Außenfläche der Platte verbindet, und das distale Ende des Hilfsmittels die Form einer Gabel (73) mit zwei Zinken (73a, 73b) aufweist, deren Innenflächen (74) ein V bilden, wobei einer der Zinken in die erste Ausnehmung (51) greifen kann.

11. Set nach Anspruch 10,
bei dem die Platte eine zweite Ausnehmung auf ihrer Außenfläche aufweist, welche eine Abschrägung begrenzt, wobei die erste und die zweite Ausnehmung ein V-Profil definieren, welches demjenigen entspricht, das durch die Gabel des distalen Endes des Hilfsinstruments gebildet wird.

## Claims

1. Fixing plate for jawbones, of elongate shape extending mainly in a longitudinal direction L, with an inner face (40) intended to come into contact with the jawbones and an opposed outer face (41, 81), said plate comprising a front side (42, 82) and a rear side (43, 83), connected by a lower side (44, 84) and an upper side (45, 85), and in which are made at least three orifices (46 to 49; 86 to 89) for means (90 to 93) for fixing in the jawbones (1, 2), at least one orifice (46, 47; 86, 87) at the rear of the plate, one orifice (48, 88) at the front of the plate and one orifice (49, 89) of oblong shape situated between said at least one rear orifice (47) and the front orifice and extending substantially in the longitudinal direction L, **characterised in that** it comprises a notch (5) which extends substantially in the longitudinal direction L and includes two substantially longitudinal walls connected by a bottom extending substantially transversely, in the front side (42, 82) of the plate or in its upper side (45, 85) between the front side to just above the rear end (490, 890) of the oblong orifice (49, 89), this notch being arranged to cooperate with the distal end of an ancillary device.

2. Plate according to claim 1, **characterised in that** the angle of inclination of said notch (5) with respect to the longitudinal direction L is between 0 and 20°.

3. Plate according to claim 1 or 2, **characterised in that** it comprises, in its inner face (40), a first recess (51) as an extension of the bottom (50) of the notch.

4. Plate according to claim 3, **characterised in that** the first recess (51) delimits a cutaway.

5. Plate according to claim 3 or 4, **characterised in that** it also comprises a second recess in the outer face (41, 81) of the plate.

6. Plate according to claim 5, **characterised in that** the second recess also delimits a cutaway, the first and second recesses defining a V-shaped profile, the bottom of the V being situated at the bottom (50) of the notch.

7. Plate according to one of claims 1 to 6, **characterised in that** it is folded, towards its inner face, about at least one fold line (60 to 63).

8. Kit including a fixing plate for jawbones (4, 8) according to claim 1 or 2 and an ancillary device (7) for positioning said plate on said bones, said ancillary device including a rigid rod (70) with a proximal end and a distal end (72), the latter being capable of being inserted in the notch (5) in the plate and including a recess for cooperating with the bottom (50) of the notch, in abutting or embedded relationship, and to become engaged with said plate.

9. Kit according to claim 8, in which the plate comprises in its inner face (40) a first recess (51) as an extension of the bottom (50) of the notch, a portion of the distal end (72) of the ancillary device being capable of being inserted in said first recess.

10. Kit according to claim 9, in which said first recess (51) delimits a cutaway (52) connecting the inner face and the outer face of the plate, and the distal end of the ancillary device is in the form of a fork (73) with two prongs (73a, 73b) of which the inner faces (74) form a V, one of the prongs being capable of being inserted in said first recess (51).

11. Kit according to claim 10, in which the plate comprises a second recess in its outer face delimiting a cutaway, the first and second recesses defining a V-shaped profile corresponding to the one formed by the fork of the distal end of the ancillary device.
